Europäisches Patentamt

European Patent Office

Office européen des brevets

(19)

(11) Veröffentlichungsnummer: **0 008 114**
**B1**

(12) ## EUROPÄISCHE PATENTSCHRIFT

(45) Veröffentlichungstag der Patentschrift:
**19.01.83**

(51) Int. Cl.³: **C 07 D 303/16, C 07 D 301/14**

(21) Anmeldenummer: **79102859.0**

(22) Anmeldetag: **08.08.79**

(54) **Verfahren zur Herstellung von Maleinsäureglycidylestern.**

(30) Priorität: **16.08.78 DE 2835883**

(43) Veröffentlichungstag der Anmeldung:
**20.02.80 Patentblatt 80/4**

(45) Bekanntmachung des Hinweises auf die Patenterteilung:
**19.01.83 Patentblatt 83/3**

(84) Benannte Vertragsstaaten:
**CH DE FR GB IT NL**

(56) Entgegenhaltungen:
**DE-A-1 942 557**
**DE-A-2 602 776**
**DE-B-1 082 263**
**DE-B-1 083 797**
**FR-A-1 269 628**

(73) Patentinhaber: **BAYER AG, Zentralbereich Patente, Marken und Lizenzen, D-5090 Leverkusen 1, Bayerwerk (DE)**

(72) Erfinder: **Rauleder, Gebhard, Dr., Mozartstrasse 20, D-5657 Haan (DE)**
Erfinder: **Waldmann, Helmut, Dr., Carl-Rumpff-Strasse 59, D-5090 Leverkusen 1 (DE)**
Erfinder: **Bottenbruch, Ludwig, Dr., Woehlerstrasse 5, D-4150 Krefeld 1 (DE)**
Erfinder: **Prater, Klaus, Dr., Bodelschwinghstrasse 22, D-4150 Krefeld 1 (DE)**
Erfinder: **Seifert, Hermann, Dr., Ruwergasse 4, D-5000 Köln 80 (DE)**
Erfinder: **Swodenk, Wolfgang, Dr., Auf dem Broich 5, D-5068 Odenthal (DE)**

## Verfahren zur Herstellung von Maleinsäureglycidylestern

Die vorliegende Erfindung betrifft ein Verfahren zur Herstellung von Maleinsäureglycidylestern aus Maleinsäurediallylester.

Maleinsäureglycidylester finden als Ausgangsprodukt zur Herstellung von Gießharzen, als Stabilisator, als Weichmacher und als Ausgangsprodukt zur Herstellung von Lacken, Klebstoffen und Kunststoffen Verwendung (s. z. B. DE-B-1 082 263, DE-A-2 023 148, GB-A-729 883, US-A-2 781 333, US-A-3 155 638 und GB-A-862 588).

Es ist bekannt, daß man Glycidylester aus Carbonsäuren, Carbonsäureanhydriden und Salzen von Carbonsäuren durch Umsetzung mit Epihalogenhydrin herstellen kann (s. z. B. DE-B-1 165 030, DE-A-1 643 777, US-A-2 448 602 und DE-A-2 023 148). Ein grundsätzlicher Nachteil dieser Verfahren besteht darin, daß salzhaltige umweltbelastende Abwässer gebildet werden.

In der GB-A-735 001 wird die Herstellung von Glycidylestern durch Umsetzen von Glycid mit Polycarbonsäurechloriden beschrieben. Dieses Verfahren hat den großen Nachteil, daß die Ausgangsmaterialien instabil und sehr reaktionsfähig sind, so daß bei der Herstellung, der Lagerung und der Verwendung dieser Verbindungen äußerste Vorsicht erforderlich ist (vgl. DE-A-2 032 148, S. 1, Z. 6).

Eine Möglichkeit, Olefine in Epoxide umzuwandeln unter Umgehung dieser Nachteile, besteht in der Anwendung der »Prileschajew-Reaktion« (vgl. N. Prileschajew, Ber. dtsch. chem. Ges. 42, 4811 [1909]). Bei dieser Reaktion handelt es sich um einen elektrophilen Angriff einer Percarbonsäure auf ein Olefin (vgl. K. D. Bingham, G. D. Meakins, G. H. Whitham, Chem. Commun. 1966, S. 445 und 446). Aus diesem Grunde nimmt die Reaktivität des Olefins mit fallender Nucleophilie der Doppelbindung ab. Deshalb erschweren elektronegative Substituenten die Epoxidation (vgl. S. N. Lewis in R. L. Augustin, »Oxidation« Vol. I, S. 227, Z. 9 – 13, Marcel Dekker, New York [1969]).

Elektronenziehende Gruppierungen wie z. B. Carboxyl, Carbonyl usw. vermindern die Reaktionsgeschwindigkeit enorm (vgl. H. Batzer und E. Nikles, Chimia, Vol. 16, Seite 62 [1962]). Insbesondere lassen sich daher Allylester nicht ohne weiteres mit Percarbonsäuren epoxidieren (vgl. DE-A-2 023 148). Infolge der geringen Reaktionsfähigkeit ihrer Doppelbindung sind hohe Temperaturen und lange Reaktionszeiten erforderlich, was zur Bildung von unerwünschten Nebenprodukten, wie Dihydroxy- und Hydroxyacyloxy-Derivaten der Ausgangsprodukte, Anlaß gibt (vgl. S. N. Lewis in R. L. Augustin. »Oxidation« Vol. 1, S. 233, Z. 6 – 11, Marcel Dekker, New York [1969]).

In zwei Patentschriften (US-A-3 155 638 und FR-A-1 394 195) wird vorgeschlagen, zur Epoxidation von äthylenisch ungesättigten Carbonsäureestern Monoperphthalsäure zu verwenden. Ein Nachteil dieser Methode ist jedoch die technisch aufwendige Rückgewinnung der Phthalsäure aus dem Reaktionsgemisch. So wird in der US-A-3 155 638 im Beispiel 12, S. 9, nach der Umsetzung von Maleinsäurediallylester mit Phthalsäureanhydrid und Wasserstoffperoxid die abgeschiedene Phthalsäure abfiltriert. Der im Filtrat verbleibende Rest muß durch wiederholtes Waschen mit Wasser und Natronlauge rückgewonnen werden und daran anschließend müssen zur Wiedergewinnung der in den wäßrigen Phasen enthaltenden Phthalsäure diese wieder angesäuert werden, was zur Bildung von unerwünschten Salzabfällen führt. Bei diesem Verfahren wird nur Allylglycidylmaleat und dieses nur in einer Ausbeute von 24% d. Th. erhalten.

Auch die Anwendung von m-Chlorperbenzoesäure zur Epoxidation der Allylester von Polycarbonsäuren wie sie in der Literatur vorgeschlagen wurde (vgl. S. R. Sandler und F. R. Berg, J. Chem. und Eng. Data, Vol. 11, S. 447 und 448 [1966]) ist für eine technische Anwendung nicht geeignet, da die vorgeschlagenen Reaktionsbedingungen von 3 bis 5°C, die Verwendung von kostspieliger Kühlsole notwendig macht und die angegebene Reaktionszeit von 3 Tagen technisch nicht akzeptabel ist. Trotz dieser aufwendigen Reaktionsbedingungen wurde beispielsweise Diglycidylterephthalat nur in einer Ausbeute von 28% erhalten (vgl. Seite 448, Zeile 13).

Über die Umsetzung von Maleinsäurediallylester mit Peressigsäure wird in J. Am. Chem. Soc. 81, S. 3354 (1959) berichtet. Trotz 4fachem Überschuß an Ester betrug die Reaktionszeit 11,5 Stunden bei 50°C. Allylglycidylmaleat wurde in einer Ausbeute von nur 71% erhalten. Die Herstellung von Diglycidylmaleat ist nicht erwähnt.

In der US-A-2 761 870 wird in den Beispielen 1 bis 5 und Beispiel 8 die Epoxidierung von Crotylestern von Carbonsäuren mit 45%iger Peressigsäure beschrieben. Auch hier sind die Reaktionsbedingungen, das Reaktionsgemisch muß 2 Tage bei 0° bis 25°C stehen, für eine technische Anwendung (Verwendung von Kühlsole, lange Reaktionszeit) äußerst aufwendig. Nach der Reaktion wird die Essigsäure durch Waschen mit 20%iger Natronlauge aus dem Reaktionsgemisch entfernt. Will man die Essigsäure wiedergewinnen, muß die wäßrige Phase wieder angesäuert werden, was zur Bildung von umweltbelastenden Salzabfällen führt.

Aus der DE-B-1 083 797 ist ein Verfahren zur Herstellung von Epoxiden durch Umsetzung von Monoolefinen mit 7 bis 12 C-Atomen mit Peressigsäure bekannt, bei dem man das Reaktionsgemisch in Gegenwart eines Überschusses an Olefin destilliert, der ausreicht, um die entstandene Essigsäure als azeotropes Gemisch zu entfernen. Diese Destillation wird bei Temperaturen unter 100°C durchgeführt, und man kann in Gegenwart eines über dem Siedepunkt des Epoxids siedenden Treibmittels arbeiten. Das erfindungsgemäße Verfahren unterscheidet sich von diesem Verfahren wesentlich. So entstehen erfindungsgemäß höher als Essigsäure siedende Carbonsäuren, von denen nicht bekannt ist, ob sie mit Maleinsäurediallylester Azeotrope bilden können. Solche Azeotrope hätten in jedem Fall aber wesentlich höhere Siedepunkte als die gemäß der DE-AS auftretenden. Außerdem kann der erfindungsgemäß einzusetzende Allylester nicht im Überschuß eingesetzt werden, weil sonst die Bildung von Diglycidylester nicht mehr möglich ist. Der erfindungsgemäß zuzufügende Zusatzstoff soll zwischen der entstehenden Carbonsäure und dem nächst höher siedenden Bestandteil sieden, nicht oberhalb des gebildeten Epoxids. Erfindungsgemäß kann die Destillation nicht auf Temperaturen unter 100°C begrenzt werden, in der DE-B-1 083 797 wird dagegen ausgesagt, daß bereits über 50°C die gebildete Carbonsäure Epoxide aufspalten kann, und erfindungsgemäß können Bis-Epoxide vorliegen, die erhöhte Angriffsmöglichkeiten haben im Vergleich zu den Monoepoxiden, die gemäß der DE-B-1 083 797 erhalten werden. Schließlich geht aus dieser Druckschrift nicht hervor, daß das dort beschriebene Verfahren zur Epoxidation von Diallylestern geeignet ist, die bekanntermaßen schwierig zu epoxidieren sind. Aus allen diesen Gründen kann dieses bekannte Verfahren das erfindungsgemäße Verfahren nicht nahelegen.

Es wurde nun ein Verfahren zur Herstellung von Maleinsäureglycidylestern gefunden, das dadurch gekennzeichnet ist, daß man Maleinsäurediallylester mit einer 3 bis 4 Kohlenstoffatome enthaltenden Percarbonsäure in Gegenwart von organischen Lösungsmitteln bei einer Temperatur von 30 bis 100°C umsetzt und anschließend das erhaltene Reaktionsgemisch durch Destillation aufarbeitet, wobei man die aus der Percarbonsäure entstandene Carbonsäure abtrennt, indem man dem Reaktionsgemisch einen Zusatzstoff zufügt, der zwischen der aus der Percarbonsäure entstehenden Carbonsäure und dem nächst höher siedenden Bestandteil des Reaktionsgemisches siedet und die Carbonsäure zusammen mit dem organischen Lösungsmittel durch Destillation abtrennt.

Der nach dem erfindungsgemäßen Verfahren einsetzbare Maleinsäurediallylester kann in an sich bekannter Weise erhalten werden, beispielsweise durch Veresterung von Maleinsäureanhydrid mit Allylalkohol.

Als organische Lösungsmittel können im erfindungsgemäßen Verfahren die verschiedensten unsubstituierten und substituierten Kohlenwasserstoffe verwendet werden, die unter Reaktionsbedingungen flüssig sind und unerwünschte Nebenreaktionen nicht oder nur in ganz untergeordnetem Maße eingehen. Als Kohlenwasserstoffe können z. B. verwendet werden aliphatische und cycloaliphatische Kohlenwasserstoffe wie Hexan, Heptan, Octan, 2-Äthyl-hexan, Decan, Dodecan, Cyclohexan, Methylcyclopentan und Petroläther, weiterhin aromatische Kohlenwasserstoffe wie Benzol, Nitrobenzol, Toluol, Äthylbenzol, Cumol, Diisopropylbenzol, Xylol und Chlorbenzol, weiterhin sauerstoffhaltige Kohlenwasserstoffe wie Diäthyläther, Diisopropyläther, Dibutyläther, Tetrahydrofuran, Dioxan, Essigsäureäthylester, Essigsäuremethylester, Essigsäurepropylester, Essigsäurebutylester, Propionsäuremethylester, Propionsäureäthylester, Propionsäurepropylester, Buttersäuremethylester, Buttersäureäthylester, Buttersäurepropylester, Buttersäurebutylester, Benzoesäuremethylester und Benzoesäureäthylester, weiterhin chlorierte Kohlenwasserstoffe wie Methylenchlorid, Chloroform, Tetrachlorkohlenstoff, 1-Chloräthan, 1,2-Dichloräthan, 1,1-Dichloräthan, 1,1,2,2-Tetrachloräthan, 1-Chlorpropan, 2-Chlorpropan, 1,2-Dichlorpropan, 1,3-Dichlorpropan, 2,3-Dichlorpropan, 1,2,3-Trichlorpropan, 1,1,2,3-Tetrachlorpropan, Butylchlorid, 1,2-Dichlorbutan, 1,4-Dichlorbutan, 2,3-Dichlorbutan, 1,3-Dichlorbutan, 1,2,3,4-Tetrachlorbutan, tert.-Butylchlorid, Amylchlorid, 1,2-Dichlorpentan, 1,5-Dichlorpentan, 1,2,3,4-Tetrachlorpentan, Cyclopentylchlorid, 1,2-Dichlorcyclopentylchlorid, Hexylchlorid, 1,2-Dichlorhexan, 1,6-Dichlorhexan, 1,2,3,4-Tetrachlorhexan, 1,2,5,6-Tetrachlorhexan, Cyclohexylchlorid, Chlorbenzol, Heptylchlorid, 1,2-Dichlorheptan, 1,2,3,4-Tetrachlorheptan, Cycloheptylchlorid, Octylchlorid, 1,2-Dichloroctan, 1,2,3,4-Tetrachloroctan und Cyclooctylchlorid.

Bevorzugte Lösungsmittel sind von den chlorierten Kohlenwasserstoffen Methylenchlorid, Chloroform, Tetrachlorkohlenstoff und 1,2-Dichlorpropan, von den aromatischen Kohlenwasserstoffen Benzol, Nitrobenzol, Toluol und Chlorbenzol, von den aliphatischen und cycloaliphatischen Kohlenwasserstoffen 2-Äthyl-hexan, Cyclohexan und Methylcyclopentan und von den sauerstoffhaltigen Kohlenwasserstoffen Tetrahydrofuran, Propionsäureäthylester und Benzoesäureäthylester.

Besonders bevorzugte Lösungsmittel sind von den chlorierten Kohlenwasserstoffen 1,2-Dichlorpropan und Tetrachlorkohlenstoff, von den aromatischen Kohlenwasserstoffen Benzol und Chlorbenzol, von den aliphatischen und cycloaliphatischen Kohlenwasserstoffen Cyclohexan und von den sauerstoffhaltigen Kohlenwasserstoffen Propionsäureäthylester und Benzoesäureäthylester.

Verwendet werden können auch Lösungsmittelgemische der verschiedenen oben angegebenen organischen Lösungsmittel.

Erfindungsgemäß verwendbare Percarbonsäuren sind Perpropionsäure, Perbuttersäure und Perisobuttersäure. Bevorzugt verwendet werden Perpropionsäure und Perisobuttersäure. Besonders bevorzugt ist Perpropionsäure. Diese Percarbonsäuren, gelöst in einem der genannten organischen Lösungsmittel, können z. B. nach dem in der DE-A-2 262 970 beschriebenen Verfahren hergestellt werden, bei dem wäßriges Wasserstoffperoxid mit der entsprechenden Carbonsäure in Gegenwart von Schwefelsäure umgesetzt und anschließend die entstandene Percarbonsäure mit einem organischen Lösungsmittel aus dem Reaktionsgemisch extrahiert wird. Gegebenenfalls kann die so erhaltene Percarbonsäurelösung in dem organischen Lösungsmittel noch weiter gereinigt werden,

3

insbesondere um den Gehalt an Wasser, Wasserstoffperoxid und Schwefelsäure zu erniedrigen.

Im allgemeinen verwendet man die Percarbonsäuren in Form einer Lösung in einem organischen Lösungsmittel. Derartige Percarbonsäurelösungen können beispielsweise 10 bis 30 Gew.-% der jeweiligen Percarbonsäure, bezogen auf die Lösung, enthalten. Die Allylester können als solche oder ebenfalls gelöst in einem oder mehreren der vorstehend genannten Lösungsmittel eingesetzt werden, wobei beliebig konzentrierte Lösungen der Allylester zum Einsatz gelangen können. Vorzugsweise werden die Allylester als solche eingesetzt und organische Lösungsmittel nur in Form der Percarbonsäurelösung zugegeben.

Das Molverhältnis von eingesetzter Percarbonsäure zu eingesetztem Maleinsäurediallylester kann in weiten Grenzen schwanken. Beispielsweise kann dieses Molverhältnis 0,1 : 1 bis 10 : 1 betragen. Bevorzugt wird ein Molverhältnis von 0,15 : 1 bis 5 : 1 angewendet. Ganz besonders vorteilhaft ist es, ein Molverhältnis von 0,2 bis 3 Mol Persäure je Mol Maleinsäurediallylester anzuwenden.

Der Wassergehalt der verwendeten Percarbonsäure soll im allgemeinen möglichst niedrig sein. Wassermengen bis 10 Gew.-% in der Percarbonsäurelösung sind im allgemeinen nicht störend. Geeignet ist beispielsweise eine Percarbonsäurelösung mit einem Wassergehalt von bis zu 2 Gew.-%. Vorzugsweise verwendet man eine Percarbonsäurelösung, die weniger als 1 Gew.-% Wasser enthält. Besonders bevorzugt ist ein Wassergehalt von weniger als 0,1 Gew.-%.

Der Wasserstoffperoxidgehalt der verwendeten Percarbonsäure in einem der obengenannten organischen Lösungsmittel soll im allgemeinen möglichst niedrig sein. Er kann beispielsweise bis zu 1 Gew.-%, bezogen auf die Percarbonsäurelösung, betragen. Vorteilhaft arbeitet man bei einem Gehalt von weniger als 0,5 Gew.-%. Besonders vorteilhaft ist es, die Umsetzung mit einer Percarbonsäurelösung durchzuführen, die einen Wasserstoffperoxidgehalt unterhalb 0,2 Gew.-% aufweist.

Der Mineralsäuregehalt der verwendeten Percarbonsäure soll möglichst niedrig sein. Vorteilhaft ist es, die Reaktion mit einer Percarbonsäurelösung durchzuführen, die einen Mineralsäuregehalt unterhalb 0,005 Gew.-% besitzt. Besonders vorteilhaft ist ein Mineralsäuregehalt von weniger als 0,001 Gew.-%.

Das erfindungsgemäße Verfahren wird in dem Temperaturbereich von 30 − 100°C durchgeführt. Bevorzugt arbeitet man bei 40 − 80°C, besonders bevorzugt bei 50 − 75°C. In Sonderfällen können die angegebenen Temperaturen auch unter- oder überschritten werden.

Neben der Arbeitsweise unter isothermen Bedingungen, d.h. Einhaltung einer einheitlichen Temperatur im gesamten Reaktionsgemisch, kann man das erfindungsgemäße Verfahren auch unter Ausbildung eines sogenannten Temperaturgradienten durchführen, der im allgemeinen mit fortschreitender Reaktion zunimmt. Man kann aber auch die Reaktion so führen, daß sich mit dem Fortschreiten der Reaktion ein Gradient fallender Temperatur ausbildet.

Das erfindungsgemäße Verfahren kann bei den verschiedensten Drucken durchgeführt werden. Im allgemeinen arbeitet man bei Normaldruck. Das Verfahren kann jedoch auch bei Unter- oder Überdruck durchgeführt werden.

Die Durchführung des erfindungsgemäßen Verfahrens kann diskontinuierlich oder kontinuierlich in den für Umsetzungen dieser Art üblichen Vorrichtungen, wie Rührwerkskessel, Siedereaktoren, Röhrenreaktoren, Schlaufenreaktoren oder Schleifenreaktoren, erfolgen.

Als Werkstoffe für die Reaktionsapparate zur Durchführung des erfindungsgemäßen Verfahrens können beispielsweise Glas, Edelstähle, oder emailliertes Material verwendet werden.

Schwermetallionen im Reaktionsgemisch katalysieren die Zersetzung der Percarbonsäure. Man setzt deshalb der Percarbonsäurelösung im allgemeinen Substanzen zu, die Schwermetallionen durch Komplexbildung inaktivieren können. Bekannte Substanzen solcher Art sind beispielsweise Gluconsäure, Äthylendiamintetraessigsäure, Natriumsilicat, Natriumpyrophosphat, Natriumhexametaphosphat, Dinatriumdimethylpyrophosphat oder $Na_2$ (2-Äthyl-hexyl)$_5$($P_3O_{10}$)$_2$ (vgl. DE-B-1 056 596, Spalte 4, Zeile 60 ff.).

Die Reaktionswärme kann durch innen- oder außenliegende Kühler abgeführt werden. Zur Ableitung der Reaktionswärme kann die Umsetzung auch unter Rückfluß (z. B. in Siedereaktoren) durchgeführt werden.

Der Allylester und die Percarbonsäure können auf beliebige Weise zusammengebracht werden. Beispielsweise kann man beide Komponenten gleichzeitig oder nacheinander in beliebiger Reihenfolge in das Reaktionsgefäß einbringen. Bei diskontinuierlicher Arbeitsweise wird vorzugsweise der Allylester vorgelegt und dann die Percarbonsäurelösung zugegeben. Die Reaktionstemperatur kann dabei vor oder nach der Zugabe der Percarbonsäure eingestellt werden. Man kann auch die beiden Komponenten bei Raumtemperatur gleichzeitig in das Reaktionsgefäß eingeben und dann die Reaktionstemperatur einstellen. Bei kontinuierlicher Arbeitsweise kann man die beiden Komponenten gemeinsam oder getrennt dem Reaktor zuführen. Bei Verwendung mehrerer Reaktoren, die beispielsweise als Kaskade hintereinander geschaltet sein können, kann es vorteilhaft sein, den Allylester nur in den ersten Reaktor einzubringen. Man kann die Allylesterzugabe jedoch auch auf mehrere Reaktoren verteilen.

Wenn als Reaktionsprodukt der Maleinsäurediglycidylester angestrebt wird, kann es von Vorteil sein, den Maleinsäurediallylester durch chargenweise Zugabe der Percarbonsäure zu epoxidieren.

4

Dabei kann es von Vorteil sein, den Maleinsäureallylglycidylester aus dem Reaktionsgemisch abzutrennen und einer erneuten Umsetzung mit Percarbonsäure zu unterwerfen.

Es kann weiterhin von Vorteil sein, die zur Erreichung des gewünschten Endproduktes (Maleinsäureallylglycidylester oder Maleinsäurediglycidylester) benötigte Menge Percarbonsäure chargenweise zuzugeben. Bei der chargenweise Zugabe der Percarbonsäure kann es auch von Vorteil sein, nach Zugabe einzelner Chargen die aus der Percarbonsäure entstandene Carbonsäure aus dem Reaktionsgemisch zu entfernen, z. B. durch Extraktion mit Wasser oder durch Destillation.

Die nach Durchführung des erfindungsgemäßen Verfahrens erhaltenen Reaktionsgemische enthalten im allgemeinen das verwendete organische Lösungsmittel, die aus der Percarbonsäure entstehende Carbonsäure Maleinsäureallylglycidylester, Maleinsäurediglycidylester und gegebenenfalls nicht umgesetzten Maleinsäurediallylester, sowie gegebenenfalls geringe Mengen von hochsiedenden Nebenprodukten. Im allgemeinen enthält das Reaktionsgemisch aus dem erfindungsgemäßen Verfahren keine Produkte, in denen die zwischen den beiden Carboxylgruppen des Maleinsäurediallylesters befindliche C = C-Doppelbindung epoxidiert worden ist.

Die Aufarbeitung des erhaltenen Reaktionsgemisches erfolgt durch Destillation. Dabei kann man so verfahren, daß man die einzelnen Komponenten in der Reihenfolge ihrer Siedepunkte abdestilliert. Dabei ist es von Vorteil, die Destillation im Vakuum und unter Verwendung von Verdampfern, die kurze Verweilzeiten und eine Verdampfung mit geringer thermischer Belastung des Reaktionsgemisches ermöglichen, durchzuführen. Erfindungsgemäß wird vor der destillativen Aufarbeitung dem Reaktionsgemisch ein geeigneter Zusatzstoff zugefügt, der zwischen der Carbonsäure und dem nächst höher siedenden Bestandteil des Reaktionsgemisches siedet. Dadurch erreicht man eine vollständige Abtrennung der Carbonsäure bei relativ niedrigen Sumpftemperaturen, was hinsichtlich der Minimierung der Bildung von Nebenprodukten aus der Carbonsäure und den epoxidierten Bestandteilen des Reaktionsgemisches von Vorteil ist. Erfindungsgemäß wird dann die Carbonsäure zusammen mit dem Lösungsmittel abdestilliert.

Es kann vorteilhaft sein, dem Reaktionsgemisch vor oder während der Reaktion und/oder vor oder während der Aufarbeitung Stabilisatoren zuzusetzen, welche die Bildung von Hochsiedern und Polymerisaten verhindern.

Das erfindungsgemäße Verfahren hat eine Reihe von Vorteilen. Mit diesem Verfahren ist es erstmals möglich, nach der sogenannten Prileschajew-Reaktion in technischem Maßstab Glycidylester von Maleinsäure herzustellen und dadurch die Nachteile des Epihalogenhydrin-Verfahrens zu vermeiden.

Gegenüber den eingangs zitierten Anwendungen der Prileschajew-Reaktion besteht ein besonderer Vorteil des erfindungsgemäßen Verfahrens darin, daß die aus der Percarbonsäure entstehende Carbonsäure durch solche Maßnahmen aus dem Reaktionsgemisch entfernt werden kann, bei denen keine umweltbelastenden salzhaltigen Abwässer anfallen. Nach dem erfindungsgemäßen Verfahren ist es möglich, sowohl Allylglycidylmaleat, als auch Diglycidylmaleat in hoher Reinheit technisch herzustellen.

Das folgende Beispiel erläutert die Erfindung. Sämtliche Prozentangaben sind, soweit nichts anderes gesagt wird, Gewichtsprozente.

## Beispiel

980 g (5 Mol) Maleinsäurediallylester wurden bei 70° mit 3600 g einer 20%igen benzolischen Perpropionsäurelösung (8 Mol), die einen Wassergehalt von unter 0,1%, einen Wasserstoffperoxidgehalt von unter 0,2% und einen Mineralsäuregehalt von unter 0,001% besaß, umgesetzt. Nachdem die Persäure weitgehend abreagiert hatte, wurde die Reaktionslösung mit 1,5 l Benzoesäuremethylester versetzt und in einer Füllkörperkolonne mit Fallstromverdampfer fraktioniert. Bei einem Vakuum von 200 mbar wurde Benzol und Propionsäure als gemeinsames Kopfprodukt erhalten, das in einer weiteren Kolonne in die Komponenten aufgetrennt wurde.

Die Redestillation des Sumpfproduktes bei 20 mbar ergab reinen Benzoesäuremethylester als Kopfprodukt und ein Sumpfprodukt, das im wesentlichen aus Maleinsäurediallylester, Maleinsäureallylglycidylester und Maleinsäurediglycidylester bestand. Dieses Sumpfprodukt wurde in einer weiteren Kolonne bei einem Vakuum von 0,2 mbar fraktioniert.

Maleinsäurediallylester und Maleinsäureallylglycidylester wurden zur erneuten Umsetzung in den Reaktor zurückgeführt.

Maleinsäurediglycidylester wurde mit einer Reinheit von 99,3% erhalten.

Kp = 148° bei 0,2 mbar

Die Elementaranalyse ergab folgende Werte:

|   | berechnet | gefunden |
|---|---|---|
| C | 52,6% | 52,6% |
| H | 5,3% | 5,4% |
| O | 42,0% | 42,0% |

**Patentansprüche**

1. Verfahren zur Herstellung von Maleinsäureglycidylestern, dadurch gekennzeichnet, daß man Maleinsäurediallylester mit einer 3 bis 4 Kohlenstoffatome enthaltenden Percarbonsäure in Gegenwart von organischen Lösungsmitteln bei einer Temperatur von 30 bis 100°C umsetzt und anschließend das erhaltene Reaktionsgemisch durch Destillation aufarbeitet, wobei man die aus der Percarbonsäure entstandene Carbonsäure abtrennt, indem man dem Reaktionsgemisch einen Zusatzstoff zufügt, der zwischen der aus der Percarbonsäure entstehenden Carbonsäure und dem nächst höher siedenden Bestandteil des Reaktionsgemisches siedet und die Carbonsäure zusammen mit dem organischen Lösungsmittel durch Destillation abtrennt.

2. Verfahren nach Anspruch 1, dadurch gekennzeichnet, daß man Perpropionsäure einsetzt.

3. Verfahren nach Anspruch 1 und 2, dadurch gekennzeichnet, daß man die Umsetzung in Gegenwart von 1,2-Dichlorpropan, Tetrachlorkohlenstoff, Benzol, Chlorbenzol, Cyclohexan und/oder Propionsäureäthylester durchführt.

4. Verfahren nach Anspruch 1 bis 3, dadurch gekennzeichnet, daß man Percarbonsäure in Form einer Lösung in einem organischen Lösungsmittel verwendet, wobei die Lösung weniger als 1 Gew.-% Wasserstoffperoxid und weniger als 0,005 Gew.-% Mineralsäure enthält.

5. Verfahren nach Anspruch 1 bis 4, dadurch gekennzeichnet, daß man 0,1 bis 10 Mol Percarbonsäure pro Mol Allylester anwendet.

6. Verfahren nach Anspruch 1 bis 5, dadurch gekennzeichnet, daß man dem Reaktionsgemisch vor oder während der Reaktion und/oder vor oder während der Aufarbeitung Stabilisatoren zusetzt, welche die Bildung von Hochsiedern und Polymerisaten verhindert.

**Claims**

1. Process for the preparation of maleic acid glycidyl esters, characterised in that maleic acid diallyl ester is reacted with a percarboxylic acid containing 3 to 4 carbon atoms in the presence of organic solvents at a temperature of 30 to 100°C and then the reaction mixture obtained is worked up by distillation, the carboxylic acid formed from the percarboxylic acid being separated off, by adding to the reaction mixture an additive which has a boiling point between that of the carboxylic acid forming from the percarboxylic acid and that of the constituent of the reaction mixture with the next highest boiling point and the carboxylic acid is separated off by distillation together with the organic solvent.

2. Process according to Claim 1, characterised in that perpropionic acid is employed.

3. Process according to Claim 1 and 2, characterised in that the reaction is carried out in the presence of 1,2-dichloropropane, carbon tetrachloride, benzene, chlorobenzene, cyclohexane and/or ethyl propionate.

4. Process according to Claim 1 to 3, characterised in that the percarboxylic acid is used in the form of a solution in an organic solvent, the solution containing less than 1% by weight of hydrogen peroxide and less than 0.005% by weight of mineral acid.

5. Process according to Claim 1 to 4, characterised in that 0.1 to 10 mols of percarboxylic acid are used per mol of allyl ester.

6. Process according to Claim 1 to 5, characterised in that stabilisers which prevents the formation of highboiling constituents and polymers are added to the reaction mixture before or during the reaction and/or before or during the working up.

**Revendications**

1. Procédé pour la fabrication d'esters glycidyliques d'acide maléique, caractérisé en ce que l'on fait réagir le maléate de diallyle avec un acide percarboxylique en $C_3-C_4$ en présence de solvants organiques à une température de 30 à 100°C et on traite ensuite par distillation le mélange de réaction

**0 008 114**

obtenu, on sépare l'acide carboxylique formé à partir de l'acide percarboxylique en ajoutant au mélange de réaction un additif qui bout entre l'acide carboxylique se formant à partir de l'acide percarboxylique et le constituant du mélange de réaction bouillant plus haut le plus proche, et on sépare par distillation l'acide carboxylique avec le solvant organique.

2. Procédé selon la revendication 1, caractérisé en ce que l'on utilise l'acide perpropionique.

3. Procédé selon les revendications 1 et 2, caractérisé en ce que l'on effectue la réaction en présence de 1,2-dichloropropane, de tétrachlorure de carbone, de benzène, de chlorobenzène, de cyclohexane et/ou de propionate d'éthyle.

4. Procédé selon les revendications 1 à 3, caractérisé en ce que l'on utilise l'acide percarboxylique sous forme d'une solution dans un solvant organique, la solution contenant moins de 1% en poids de peroxyde d'hydrogène et moins de 0,005% en poids d'acide minéral.

5. Procédé selon les revendications 1 à 4, caractérisé en ce que l'on utilise 0,1 à 10 moles d'acide percarboxylique par mole d'ester allylique.

6. Procédé selon les revendications 1 à 5, caractérisé en ce que l'on ajoute au mélange de réaction, avant ou pendant la réaction et/ou avant ou pendant le traitement, des stabilisants qui empêchent la formation de produits de haut point d'ébullition et de polymères.

7